# EUROPÄISCHE PATENTSCHRIFT

(11) **EP 2 854 553 B1**
(45) Veröffentlichungstag und Bekanntmachung des Hinweises auf die Patenterteilung: **21.12.2016**
(21) Anmeldenummer: 13719034.4
(22) Anmeldetag: 28.03.2013
(51) Int. Cl.: A01N 43/16, A01N 25/02, A01N 25/22, A61K 8/49, A61K 8/73, A61Q 17/00

(54) **VERWENDUNG VON DELPHINIDIN GEGEN STAPHYLOCOCCUS AUREUS**
USE OF DELPHINIDIN AGAINST STAPHYLOCOCCUS AUREUS
EMPLOI DE LA DELPHINIDINE CONTRE LE STAPHYLOCCOUS AUREUS

(30) Priorität: 30.03.2012 EP 12002352
(43) Veröffentlichungstag der Anmeldung: 08.04.2015
(73) Patentinhaber: SapioTec GmbH, 97082 Würzburg (DE)
(72) Erfinder: ROEWER, Norbert, 97082 Würzburg (DE); BROSCHEIT, Jens, 97209 Würzburg (DE)
(74) Vertreter: Glawe, Delfs, Moll
(86) Internationale Anmeldenummer: PCT/EP2013/056725
(87) Internationale Veröffentlichungsnummer: WO 2013/144306

(56) Entgegenhaltungen:
- EP-A1- 0 390 496
- "Biofiles: Nutrition Research", , 1. Februar 2007 (2007-02-01), Seiten 1-28, XP55064035, St. Louis, MO, U.S.A. Gefunden im Internet: URL:http://www.sigmaaldrich.com/etc/medial ib/docs/Sigma/General_Information/nutritio n_biofiles7.Par.0001.File.tmp/nutrition_bi ofiles7.pdf [gefunden am 2013-05-24]
- Tiurlan F Hutajalu ET AL: "Identification of Phenol and Delphinidine in the Telangs flower (Clitoria ternatea L.) and its Effectivity to Staphylococcus aureus as Eyes Bacterial Disease", Journal of Agro-Based Industry (Warta IHP Nalai Besar Industri Agro), 1. Dezember 2008 (2008-12-01), Seite 1, XP55064065, Indonesia Gefunden im Internet: URL:http://bbia.go.id/files/WartaIHPvol%20 25%20no.2-2008.pdf [gefunden am 2013-05-24]
- DEIVIDAS BURDULIS ET AL: "COMPARATIVE STUDY OF ANTHOCYANIN COMPOSITION, ANTIMICROBIAL AND ANTIOXIDANT ACTIVITY IN BILBERRY (VACCINIUM MYRTILLUS L.) AND BLUEBERRY (VACCINIUM CORYMBOSUM L.) FRUITS", ACTA POLONIAE PHARMACEUTICA, Bd. 66, Nr. 4, 1. April 2009 (2009-04-01), Seiten 399-408, XP55041258, ISSN: 0001-6837
- M I Ibrahium: "Efficiency of Pomegranate Peel Extract as Antimicrobial, Antioxidant and Protective Agents", World Journal of Agricultural Sciences, 1. April 2010 (2010-04-01), Seiten 338-344, XP55064031, Gefunden im Internet: URL:http://www.idosi.org/wjas/wjas6(4)/1.p df [gefunden am 2013-05-24]
- Sh Abdollahzadeh ET AL: "Antibacterial and Antifungal Activities of Punica Granatum Peel Extracts Against Oral Pathogens", Journal of Dentistry, 1. April 2011 (2011-04-01), Seiten 1-6, XP55064203, Tehran, Iran Gefunden im Internet: URL:http://jdt.tums.ac.ir/index.php/jdt/ar ticle/download/251/245 [gefunden am 2013-05-27]
- Pius O Ukoha ET AL: "Tannins and other phytochemical of the Samanaea saman pods and their antimicrobial activities", African Journal of Pure and Applied Chemistry, 1. August 2011 (2011-08-01), Seiten 237-244, XP55064029, Gefunden im Internet: URL:http://www.academicjournals.org/ajpac/ pdf/pdf2011/Aug/Ukoha et al.pdf [gefunden am 2013-05-24]
- FARRUKH AFAQ ET AL: "Delphinidin, an anthocyanidin in pigmented fruits and vegetables, protects human HaCaT keratinocytes and mouse skin against UVB-mediated oxidative stress and apoptosis", JOURNAL OF INVESTIGATIVE DERMATOLOGY, NATURE PUBLISHING GROUP, GB, Bd. 127, Nr. 1, 1. Januar 2007 (2007-01-01), Seiten 222-232, XP002692596, ISSN: 0022-202X, DOI: 10.1038/SJ.JID.5700510 [gefunden am 2006-08-10]
- HIGUCHI T ET AL: "Pharmaceutical composition for treating drug resistant bacterial infectious disease caused by methicillin-resistant staphylococcus aureus, contains flavonoid, benzal coumaranone and anthocyanidin as active ingredient", WPI / THOMSON,, Bd. 2003, Nr. 77, 17. Juni 2003 (2003-06-17), XP002685441, -& JP 2003 171274 A (HIGUCHI TOMIHIKO; ALPS YAKUHIN KOGYO KK) 17. Juni 2003 (2003-06-17)

## Beschreibung

Die Erfindung betrifft die Verwendung des Anthocyanidins Delphinidin oder dessen Salze zur Bekämpfung von Antibiotika-resistenten und Antibiotika-sensitiven Bakterien.

Anthocyanidine sind zymochrome Farbstoffe, die in den meisten höheren Landpflanzen vorkommen. Anthocyanidine sind zuckerfrei (Aglycone) und eng verwandt mit den zuckerhaltigen Anthocyanen (Glycoside), die beide unter den Oberbegriff der Anthocyane fallen. Anthocyanidine sind Farbstoffe und besitzen antioxidative Eigenschaften.

Antibiotika-resistente und Antibiotika-sensitive Bakterien finden sich häufig dort, wo ständig Antibiotika eingesetzt werden. Antibiotika-resistente und Antibiotika-sensitive Bakterien der Art *Staphylococcus aureus* zählen zu den gefährlichsten Erregern von im Krankenhaus erworbenen, sogenannten nosokomialen Infektionen, die sowohl über die Haut und Schleimhäute, aber auch durch kontaminierte medizinische Geräte oder Lebensmittel aufgenommen werden.

Insbesondere aufgrund des vermehrten Verbrauchs von Breitbandspektrum-Antibiotika in der Behandlung von bakteriellen Infektionskrankheiten, ist eine Zunahme von multiresistenten Bakterien zu verzeichnen, so dass bei der Behandlung von mit diesen Bakterien infizierten Patienten oder Tieren kein wirksames Antibiotikum mehr zur Verfügung steht. Antibiotika-resistente Bakterien können bei Mensch und Tier schwere Allgemeininfektionen auslösen. Dies trifft insbesondere bei immunsupprimierten Patienten zu.

Aufgabe der vorliegenden Erfindung ist es, ein wirksames Mittel zur Prophylaxe und Behandlung von bakteriellen Infektionskrankheiten zur Verfügung zu stellen.

Der Erfindung liegt die weitere Aufgabe zu Grunde, ein wirksames Mittel zur Bekämpfung von Antibiotika-resistenten und Antibiotika-sensitiven Bakterien der Art *Staphylococcus aureus* zur Verfügung zu stellen.

Gelöst werden diese Aufgaben durch die in den beigefügten Ansprüchen beanspruchten Ausführungsformen der Erfindung. Ausführungsformen in vorliegender Beschreibung, die nicht von den beigefügten Ansprüchen erfasst werden, dienen allein illustrativen Zwecken und sind nicht Gegenstand vorliegender Erfindung.

In Bezug auf Stand der Technik zu vorliegender Anmeldung sei folgendes angemerkt:
Higuchi und Kollegen (Higuchi et al., "Pharmaceutical composition for treating drug resistant bacterial infectious disease caused by methicillin-resistant staphylococcus aureus, contains flavonoid, benzal coumaranone and anthocyanidin as active ingredient", WPI / THOMSON, Bd. 2003, Nr. 77, 17 Juni 2003) beschreiben die Verwendung von Cyanidin bzw. Pelargonidin zur zumindest teilweisen Bekämpfung von *Staphylococcus aureus* auf einem Objekt, welches kein lebendes Tier ist. Im Unterschied dazu findet bei vorliegender Erfindung gemäß den beigefügten Ansprüchen das Anthocyanidin Delphinidin Verwendung. Aus dem Stand der Technik ist dem Fachmann bekannt, dass Extrakte verschiedener Früchte, welche eine Wirkung gegen *Staphylococcus aureus* zeigen, neben weiteren Anthocyanidinen auch Delphinidin enthalten. Siehe dazu beispielsweise Deividas Burdulis und Kollegen (Deividas Burdulis et al., "COMPARATIVE STUDY OF ANTHOCYANIN COMPOSITION, ANTIMICROBIAL AND ANTIOXIDANT ACTIVITY IN BILBERRY (VACCINIUM MYRTILLUS L.) AND BLUEBERRY (VACCINIUM CORYMBOSUM L.) FRUITS", ACTA POLONIAE PHARMACEUTICA, Bd. 66, Nr. 4, 1. April 2009, Seiten 399-408), Ibrahium (M I Ibrahium, "Efficiency of Pomegranate Peel Extract as Antimicrobial, Antioxidant and Protective Agents", World Journal of Agricultural Sciences, 1. April 2010, Seiten 338-344), Abdollahzadeh und Kollegen (Sh Abdollahzadeh et al., "Antibacterial and Antifungal Activities of Punica Granatum Peel Extracts Against Oral Pathogens", Journal of Dentistry, 1. April 2011, Seiten 1-6) und Ukoha und Kollegen (Pius O Ukoha et al., "Tannins and other phytochemical of the Samanaea saman pods and their antimicrobial activities", African Journal of Pure and Applied Chemistry, 1. August 2011, Seiten 237-244). Das jedoch das Anthocyanidin Delphinidin eine Wirkung gegen *Staphylococcus aureus* bewirkt war für den Fachmann mit Blick auf die Publikation von Hutajalu und Kollegen (Tiurlan F Hutajalu et al., "Identification of Phenol and Delphinidine in the Telangs flower (Clitoria ternatea L.) and its Effectivity to Staphylococcus aureus as Eyes Bacterial Disease", Journal of Agro-Based Industry (Warta IHP Nalai Besar Industri Agro), 1. Dezember 2008, Seite 1) nicht zu erwarten, in der darüber informiert wird, dass Delphinidin lediglich als blaues Pigment fungiert, nicht jedoch zu einem antimikrobiellen Effekt gegen *Staphylococcus aureus* beiträgt.

Zunächst seien einige im Rahmen der Erfindung verwendete Begriffe erläutert.

"Antibiotika" sind Arzneistoffe zur Behandlung bakterieller Infektionskrankheiten. Die Gruppe der Antibiotika umfasst insbesondere die β-Lactam Antibiotika (einschließlich Penicillin, Cephalosporine, Carbapeneme und Monobactame), Chinolone, Tetracycline, Aminoglykoside, Erythromycin, Sulfonamide und Vancomycin. "Antibiotika-resistentes Bakterium" und "Antibiotika-sensitives Bakterium" bedeutet, dass das Bakterium gegen mindestens ein Antibiotikum resistent bzw. gegenüber mindestens einem Antibiotikum nur noch verminderte Empfindlichkeit aufweist. Multiresistente *Staphylococcus aureus* Stämme, die gegen alle derzeit marktverfügbaren β-Lactam Antibiotika resistent sind und meist auch Resistenzen gegenüber anderen Antibiotikaklassen verfügen, so gegen Chinolone, Tetracycline, Aminoglykoside, Erythromycin und Sulfonamide, werden im Gebrauch unter der Bezeichnung "MRSA" (multiresistenter *Staphyloccocus aureus bzw.* Methicillinresistenter *Staphyloccocus aureus*) zusammengefasst. Eine Therapie von MRSA Patienten erfolgt üblicherweise mit dem Reserveantibiotikum Vancomycin, wobei auch dagegen schon Resistenzen verbreitet sind ("VRSA" - Vancomycin-resistenter *Staphyloccocus aureus*) und folglich ein erheblicher Bedarf an alternativen Arzneistoffen zur Behandlung bakterieller Infektionskrankheiten besteht.

Erfindungsgemäß wird die in den Ansprüchen definierte Zusammensetzung oder der Komplex oder die in den Ansprüchen definierte wässrige Lösung oder der Feststoff zur Behandlung eines Objekts oder Individuums eingesetzt, das verdächtigt wird mit Bakterien ausgewählt aus der Gruppe bestehend aus Antibiotika-resistenten *Staphylococcus aureus* und Antibiotika-sensitiven *Staphylococcus aureus* kontaminiert zu sein. Der Zweck dieser Behandlung ist die zumindest teilweise Neutralisierung der Bakterien. Die Neutralisierung umfasst z.B. bakteriostatische Neutralisierung, bakterizidische Neutralisierung, gemischte bakteriostatische-bakterizidische Neutralisierung. Der Begriff "Zusammensetzung oder Komplex umfassend mindestens ein Anthocyanidin" schließt ein Anthocyanidin als solches ohne weiteren Komponenten ein.

Mit Blick auf die Anwendung im Gesundheitssektor dient die in den Ansprüchen definierte erfindungsgemäße Zusammensetzung oder der Komplex oder die in den Ansprüchen definierte wässrige Lösung oder der Feststoff zur Verwendung in der Behandlung oder Prophylaxe von Menschen oder Tieren, die verdächtig sind, mit Antibiotika-resistenten oder Antibiotika-sensitiven Bakterien der Art *Staphylococcus aureus* infiziert zu sein. Die bakterielle Infektion bedingt insbesondere Krankheiten mit Symptomen wie Hautinfektionen, einschließlich Furunkel und Karbunkel, Pyomyositis, Lungenentzündung, Endokarditis, Toxisches Schock-Syndrom, Sepsis und Mastitis. Vor allem bei immunsupprimierten Patienten können durch Antibiotika-resistente oder Antibiotika-sensitive Bakterien der Art *Staphylococcus aureus* schwere Allgemeininfektionen ausgelöst werden.

Mit Blick auf den Nahrungs-, Futtermittel und Desinfektionssektor umfasst der Begriff "Objekt" keine lebenden Tiere, Vorrichtungen und/oder Einrichtungen und/oder Ausrüstungen und/oder Geräte für medizinische Anwendungen, die Lebensmittelverarbeitung, das Militär, Schutzausrüstungen, häusliche Objekte, Gebäudeeinrichtungen und Bauwerke. "Keine lebenden Tiere" sind insbesondere getötete geschlachtete Tiere oder Teile dieser Tiere, beispielsweise der Körper eines Rindes oder ein Teil davon, der Körper eines Schweins oder ein Teil davon, ein Geflügelkörper oder ein Teil davon und der Körper eines im Wasser lebenden Tieres oder ein Teil davon. Das "Objekt" kann insbesondere ein Nahrungs- und/oder Futtermittel sein, beispielsweise ein Fleischprodukt, ein verarbeitetes Fleischprodukt, ein Molkereiprodukt, Gemüse und deren Teile und Obst und deren Teile.

"Neutralisation" bedeutet im Sinne der vorliegenden Erfindung die Zerstörung oder Auflösung (Lysis) oder Inaktivierung (z.B. der Verlust des Infektionspotentials) oder Verhinderung der Vermehrung eines Pathogens oder die Verhinderung der Pathogen-vermittelten Biofilm-Bildung, insbesondere Antibiotika-resistenter oder Antibiotika-sensitiver Bakterien der Art *Staphylococcus aureus.* Die Verabreichung oder Applikation der erfindungsgemäßen Zusammensetzung oder des erfindungemäßen Komplexes kann derart erfolgen, dass ein Aufsprühen, Pudern, eine Injektion, eine Beschichtung mittels eines Gels, eines Verbandes, Pflasters oder ähnlichem auf den verdächtigerweise infizierten Bereich erfolgt. Bei der Behandlung von Menschen oder Tieren kann in Abhängigkeit von dem Krankheitsbild eine systemische oder lokale Verabreichung der erfindungsgemäßen

Zusammensetzung oder des erfindungsgemäßen Komplexes erfolgen. Entsprechende Techniken für die Formulierung und Verabreichung sind aus dem Stand der Technik bekannt, siehe beispielsweise "Remington's Pharmaceutical Sciences" (Mack Publishing Co, Easton Pa.). Zum Beispiel können die erfindungsgemäßen Zusammensetzungen und Komplexe einem Subjekt intravenös mittels eines pharmazeutisch akzeptablen Trägers (z.B. physiologische Salzlösung) verabreicht werden. Für die Injektion bietet sich eine Formulierung in wässriger Lösung, vorzugsweise in physiologisch akzeptablen Puffern an (z.B. Hanks-Lösung, Ringer-Lösung oder physiologisch gepufferte Salzlösung). Für die parenterale Verabreichung, einschließlich der intravenösen, subkutanen, intramuskulären und intraperitonealen Verabreichung, kommt ebenfalls eine wässrige oder ölige Lösung oder eine Feststoffformulierung in Betracht.

"Biofilm" ist eine Ansammlung von Mikroorganismen (z.B. Bakterien), eingebettet in einer von den Mikroorganismen produzierten extrazellulären Polysaccharid- oder Protein-Matrix auf einer Oberfläche. Die Organisation von Bakterien in einem Biofilm führt zu einer deutlich erhöhten Widerstandsfähigkeit der gesamten Population gegenüber verschiedensten Einflüssen. Durch Bakterien verursachte Biofilme auf Zähnen, dem Zahnfleisch, den Harnwegen, dem Verdauungstrakt oder medizinischen Geräten wie Katheter und Prothesen führen häufig zu schwerwiegenden Infektionen (Costerton et al., Annu Rev. Microbiol 1995; 49: 711-45).

"Salz" oder "pharmazeutisch akzeptables Salz" steht für jegliches unter pharmazeutischen Gesichtspunkten akzeptables Salz einer Verbindung vorliegender Erfindung, welches den pharmazeutisch wirksamen Wirkstoff oder dessen aktives Metabolit nach der Verabreichung freigeben kann. Salze der Zusammensetzungen und Komplexe der vorliegenden Erfindung können von anorganischen oder organischen Säuren und Basen abgeleitet sein.

Das Anthocyanidin, kann in "Reinform" oder "gereinigt" verwendet werden, was bedeutet, dass ungewünschte Komponenten entfernt wurden.
"Anthocyanidine" weisen die nachfolgend wiedergegebene Grundstruktur auf.

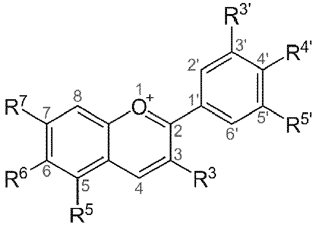

Die Substituenten in dieser Formel sind ausgewählt aus der Gruppe bestehend aus Wasserstoff, Hydroxygruppe und Methoxygruppe.

Das erfindungsgemäss verwendete Anthocyanidin ist Delphinidin. Cyclodextrine, die mit dem Anthocyanidin Delphinidin erfindungsgemäß komplexiert sein können, sind zyklische Oligosaccharide aus α-1,4-glykosidisch verknüpften Glukosemolekülen. ß-Cyclodextrin besitzt sieben Glukoseeinheiten. Bei einem Sulfoalkyether-ß-Cyclodextrin sind Hydroxygruppen der Glukoseeinheit in einem Sulfoalkylalkohol verethert. Erfindungsgemäß ist in der Regel lediglich ein Teil der 21 Hydroxygruppen eines β-Cyclodextrins verethert. Die Herstellung von Sulfoalkyethercyclodextrinen ist dem Fachmann geläufig und beispielsweise in US 5,134,127 oder WO 2009/134347 A2 beschrieben.

Sulfoalkyethergruppen werden bei Cyclodextrinen im Stand der Technik zur Erhöhung der Hydrophilie beziehungsweise Wasserlöslichkeit eingesetzt. Die Erfindung hat erkannt, dass die Sulfoalkylethergruppen in besonderem Maße zur Erhöhung der Stabilität des Komplexes aus Anthocyanidinen und dementsprechend substituierten ß-Cyclodextrin beitragen und so die Lagerstabilität und Formulierbarkeit der besonders oxidationsempfindlichen Anthocyanidine wesentlich verbessern. Der erfindungsgemäße Komplex kann als lagerstabile wässrige Lösung oder Feststoff formuliert werden, wie unten noch näher gezeigt werden wird.

Erfindungsgemäß besonders bevorzugt ist die Komplexierung mit einem Sulfobutylether-ß-Cyclodextrin (SEB-ß-CD). Ein den Schutzbereich nicht beschränkender Erklärungsversuch hierfür ist, dass die negativ geladenen Sulfobutyleinheiten mit den positiv geladenen Anthocyanidinen elektrostatisch wechselwirken und unter den Alkylgruppen die Butylgruppe die optimale Länge besitzt, um sterisch eine entsprechende Wechselwirkung zu ermöglichen.

Bevorzugt beträgt der Substitutionsgrad des Cyclodextrins mit Sulfoalkylethergruppen 3-8, weiter vorzugsweise 4-8, weiter vorzugsweise 5 bis 8, weiter vorzugsweise 6 bis 7. Geeignete Sulfobutylether-ß-Cyclodextrine mit einem mittleren Substitutionsgrad von 6 bis 7 sind beispielsweise in der genannten WO 2009/134347 A2 beschrieben und unter dem Handelsnamen Captisol^{®} kommerziell erhältlich. Ebenfalls verwendbar sind entsprechende Cyclodextrine mit einem Substitutionsgrad von 4-5, beispielsweise 4,2.

Die Anthocyanidine aus der Gruppe bestehend aus Aurantinidin, Cyanidin, Delphinidin, Europinidin, Luteolinidin, Pelargonidin, Malvidin, Peonidin, Petunidin und Rosinidin können in reiner Salz- oder komplexierter Form verwendet werden. Die chemische Struktur entspricht der oben wiedergegebenen Formel I mit dem folgenden Substitutionsmuster

| | R^{3'} | R^{4'} | R^{5'} | R³ | R⁵ | R⁶ | R⁷ |
|---|---|---|---|---|---|---|---|
| Aurantinidin | -H | -OH | -H | -OH | -OH | -OH | -OH |
| Cyanidin | -OH | -OH | -H | -OH | -OH | -H | -OH |
| Delphinidin | -OH | -OH | -OH | -OH | -OH | -H | -OH |
| Europinidin | -OCH₃ | -OH | -OH | -OH | -OCH₃ | -H | -OH |
| Luteolinidin | -OH | -OH | -H | -OH | -OH | -H | -OH |
| Pelargonidin | -H | -OH | -H | -OH | -OH | -H | -OH |
| Malvidin | -OCH₃ | -OH | -OCH₃ | -OH | -OH | -H | -OH |
| Peonidin | -OCH₃ | -OH | -H | -OH | -OH | -H | -OH |
| Petunidin | -OH | -OH | -OCH₃ | -OH | -OH | -H | -OH |
| Rosinidin | -OCH₃ | -OH | -H | -OH | -OH | -H | -OCH₃ |

Im Rahmen der Erfindung wird Delphinidin verwendet. Gegenstand der Erfindung ist ferner die anspruchsgemäße Verwendung einer wässrigen Lösung der erfindungsgemäßen Zusammensetzung oder des erfindungsgemäßen Komplexes.

Der erfindungsgemäße Komplex sowie eine entsprechende wässrige Lösung umfasst die folgenden Schritte:
a) Herstellen einer wässrigen Lösung des Sufoalkylether-ß-Cyclodextrins,
b) Zugabe des Anthocyanidins Delphinidin und Vermischen zur Herstellung des Komplexes.

In Schritt a) wird bevorzugt eine wässrige Lösung hergestellt, die 5 bis 10 Gew.-% des verwendeten Cyclodextrins enthält. Besonders bevorzugt ist es im Rahmen der Erfindung, wenn der pH-Wert der wässrigen Lösung während oder nach, bevorzugt jedoch vor der Zugabe des Anthocyanidins (Delphinidin), auf einen pH-Wert von 7 oder weniger, vorzugsweise 6 oder weniger, weiter vorzugsweise 5 oder weniger, weiter vorzugsweise 4 bis 5, eingestellt wird. Es hat sich gezeigt, dass bei diesem pH-Wert sich eine höhere Konzentration des Komplexes in wässriger Lösung einstellen lässt.

Die Konzentration des Anthocyanidins berechnet als Chlorid, beträgt vorzugsweise wenigstens 0,5 mg/ml, weiter vorzugsweise wenigstens 1,0 mg/ml, weiter vorzugsweise wenigstens 1,5 mg/ml, weiter vorzugsweise 2,0 mg/ml beträgt. Der besonders bevorzugte Konzentrationsbereich von wenigstens 2,0 mg/ml lässt sich im Rahmen einer bevorzugten Ausführungsform insbesondere einstellen in einer wässrigen Lösung mit einem pH-Wert zwischen 4 und 5.

Im Rahmen der erfindungsgemäßen Herstellung kann das Vermischen der Bestandteile der wässrigen Lösung durch Rühren geschehen, bevorzugte Zeiträume für das Vermischen sind 2 bis 20 h. Bevorzugt wird im Dunkeln gearbeitet, um eine lichtinduzierte Oxidation zu vermeiden.

Ein weiterer Gegenstand der Erfindung ist die anspruchsgemäße Verwendung eines Feststoffes enthaltend einen erfindungsgemäßen Komplex, der erfindungsgemäß erhältlich ist durch Entfernen des Lösungsmittels aus einer erfindungsgemäßen wässrigen Lösung. Das Entfernen kann bevorzugt durch Gefriertrocknung (Lyophilisation) erfolgen. Sowohl die erfindungsgemäße wässrige Lösung als auch der Feststoff besitzen eine hohe Lagerstabilität.

Die Erfindung soll nun im Folgenden weiter in den Beispielen unter Bezug auf die beigefügten Figuren beschrieben werden.

### I. Herstellung eines Komplexes aus dem Anthocyanidin Delphinidin und Cyclodextrinen

### 1. Eingesetzte Materialien:

Die nachfolgenden Cyclodextrine werden verwendet:

| | |
|---|---|
| α-CD | ID No: CYL-2322 |
| β-CD | ID No: CYL-3190 |
| γ-CD | ID No: CYL-2323 |
| (2-Hydroxypropyl)-ß-CD | ID No: L-043/07 |
| Sulfobutylether-ß-CD | ID No: 47K010111 |

Delphinidinchlorid wurde von der Firma Extrasynthese erworben.

### 2. Bestimmung des Delphinidingehalts

Zur Bestimmung des Gehalts von Delphinidinchlorid in den delphinidinhaltigen Zusammensetzungen wurde ein Reversephase HPLC-Verfahren verwendet. Dabei wurden folgende Reagenzien eingesetzt:
Gereinigtes Wasser
Methanol für die Chromatographie
Armeisensäure, p. a.
1 M Salzsäure als volumetrische Lösung.

Als Säule wurde eine Waters X Bridge™ C18,35 µl, 150 mm x 4,6 mm verwendet.

Die mobilen Phasen waren wie folgt:
Kanal A: Wasser 950 ml, Methanol 50 ml, Ameisensäure 10 ml
Kanal B: Wasser 50 ml, Methanol 950 ml, Ameisensäure 10 ml

**Folgendes Gradientenprogramm wurde verwendet:**

| Zeit [min] | Prozent Kanal B |
|---|---|
| 0 | 0 |
| 5 | 0 |
| 25 | 60 |
| 30 | 100 |

Stoppzeit: 35 min
Nachlaufzeit (posttime): 8 min
Flussrate: 1 ml/min
Injektionsvolumen: 20 µl
Säulentemperatur: 30°C +/- 2°C
UV-Vis Detektor: 530 µm für den Assay, 275 µm zur Detektion von Verunreinigungen
Integrator: Fläche

Lösungen und Probenvorbereitung:
Verdünnungslösung 1: Mischung aus 100 ml Methanol und 2,6 ml 1 M HCL
Verdünnungslösung 2: Mischung aus 100 ml 40 prozentiger Methanol und 2,6 ml 1 M HCL

Kalibrierungslösung: Eine Referenzlösung von Delphinidin wurde durch Einwiegen von 10 mg Delphinidinchlorid in einen 10 ml Kolben und Lösen in Verdünnungslösung 1 hergestellt. Nach dem Lösen wurde ungefähr 10fach verdünnt mit Verdünnungslösung 2 zur Herstellung einer ungefähren Konzentration von 0,1 mg/ml.

Die Kontrollkalibrierungslösung wurde auf die gleiche Art und Weise hergestellt. Die Kalibrierungslösungen wurden sofort mittels HPLC analysiert, da Delphinidinchlorid in Lösung instabil ist.

### Herstellung der Prüflösungen:

Zur Bestimmung des Delphinidingehalts erfindungsgemäß hergestellter Feststoffe (Herstellung siehe weiter unten) wurde etwa 50 mg dieser Zusammensetzung in einem 10 ml Kolben eingewogen. Anschließend wurde in Verdünnungslösung 2 gelöst und mit der gleichen Verdünnungslösung 2 weiter verdünnt bis zur Einstellung einer ungefähren Delphinidinkonzentration von 0,1 mg/ml.

Die Bestimmung des Delphinidingehalts in den Proben wurde berechnet unter Zuhilfenahme der Agilent ChemStation Software unter Verwendung der Kalibrierung mit dem beschriebenen externen Standard.

### Beispiel 1: Komplexierung von Delphinidin mit SBE-ß-CD

In diesem Beispiel wird die Komplexierung von Delphinidin durch verschiedene Cyclodextrine und die Löslichkeit des Komplexes in wässriger Lösung untersucht.

Es wurden neutrale wässrige Lösungen hergestellt, die 10 Gew.-% des jeweiligen Cyclodextrins enthalten. Bei ß-CD wurde auf Grund der mangelnden Löslichkeit eine Konzentration von lediglich 2 Gew.-% gewählt.

Je 5 ml der wässrigen Cyclodextrinlösungen und von reinem Wasser wurden in Glaskolben gefüllt. Anschließend wurde ein Überschuss Delphinidinchlorid zugegeben. Die erforderliche Überschussmenge war 10 mg für die Lösungen von α-, ß- und γ-Cyclodextrin und 15 mg für die Lösungen von HPBCD (2-Hydroxypropyl-ß-Cyclodextrin) und SBE-ß-CD.

Die Suspensionen wurden für 20 h bei 30° C im Dunkeln gerührt. Anschließend wurde filtriert durch einen Membranfilter mit 0,22 µm Porengröße.

Die erzielbaren Löslichkeiten sind in der nachfolgenden Tabelle 1 wiedergegeben.

| Cyclodextrin | Cyclodextrin-Konzentration | Delphinidinchlorid |
|---|---|---|
| - | 0 | 0.07 mg/ml |
| α-CD | 10 % | 0.14 mg/ml |
| β-CD | 2 % | 0.05 mg/ml |
| γ-CD | 10 % | 0.21 mg/ml |
| HPBCD | 10 % | 0.19 mg/ml |
| SBE-ß-CD | 10 % | 0.66 mg/ml |

Man erkennt, dass die Komplexierung und dadurch bewirkte Löslichkeitserhöhung für SBE-ß-CD weit besser ist als für die anderen Cyclodextrine.

### Beispiel 2: Einfluss des pH-Wertes

In diesem Beispiel wurde der Einfluss des pH-Wertes auf die Löslichkeit eines Delphinidin-SBE-ß-CD in wässriger Lösung untersucht. Nach der Vorschrift von Beispiel 1 wurden wässrige Lösungen von SEB-ß-CD hergestellt, diese Lösungen jedoch mit 1 M HCL auf die in Tabelle 2 genannten sauren pH-Werte eingestellt. Anschließend wurde Delphinidinchlorid nach der Vorschrift des Beispiels 1 zugegeben und weitergearbeitet, als einzige Abweichung wurde die Rührzeit auf 2,5 h begrenzt. Die Ergebnisse sind in der nachfolgenden Tabelle 2 wiedergegeben.

| pH | Delphinidinchlorid |
|---|---|
| 6.0 | 0.60 mg/ml |
| 4.8 | 2.12 mg/ml |
| 4.1 | 2.03 mg/ml |

Man erkennt, dass bei pH-Werten zwischen 4 und 5 die Löslichkeit des komplexierten Delphinidinchlorids sich etwa um den Faktor 3 gegenüber einem neutralen pH-Wert erhöht.

### Beispiel 3 Herstellung eines erfindungsgemäßen Feststoffs

In diesem Beispiel wird ein erfindungsgemäßer Komplex als Feststoff formuliert. Zu Vergleichszwecken werden ein Delphinidin/HPBCD Komplex sowie eine Delphinidin/Stärkeformulierung als Festsoff zubereitet.

### Beispiel 3.1: Delphinidin/SBE-ß-CD

5 g SEB-ß-CD wurden in 40 ml destilliertem Wasser zu einer klaren Lösung gelöst. Der pH-Wert der Lösung wurde mittels 1 M HCL auf 4,8 eingestellt. Anschließend wurden 0,11 g Delphinidinchlorid hinzugefügt und 2 h bei 27°C im Dunkeln gerührt. Die homogene Flüssigkeit wurde durch einen Cellulosenitratmembranfilter mit einer Porengröße von 0,45 µm vakuumfiltriert. Die Lösung wurde gefroren und anschließend gefriergetrocknet bei -48°C und einem Druck von etwa 10,3 Pa (77 mTorr). Das Lyophilisat wurde gemahlen und durch ein Sieb von 0,3 mm Maschenweite gesiebt.

### Beispiel 3.2: Delphinidin/HPBCD

Es wurde in gleicher Weise wie Beispiel 3.1 gearbeitet, jedoch wurde bei der Filtration eine signifikante Menge Material abfiltriert, was darauf hindeutet, dass die Solubilisierung deutlich weniger effektiv war als bei Verwendung von SBE-ß-CD gemäß Beispiel 3.1.

### Beispiel 3.3 Delphinidin/Stärkeformulierung

5 g Stärke wurde in 40 ml destilliertem Wasser suspendiert. Man erhielt eine weiße Suspension. Der pH der Lösung wurde mit 1 M HCL auf 4,6 eingestellt. Anschließend wurde 0,11 g Delphinidinchlorid zugegeben und für 2 h bei 27° C im Dunkeln gerührt. Die erhaltene homogene Flüssigkeit wurde wie in Beispiel 3.1 gefriergetrocknet, gemahlen und gesiebt. Beispiel 3.1 ist erfindungsgemäß, bei den Beispielen 3.2 und 3.3 handelt es sich um Vergleichsbeispiele.

### Beispiel 4: Stabilitätsversuche

Die Feststoffe gemäß den Beispielen 3.1 bis 3.3 wurden unter folgenden Bedingungen gelagert:
- 8 Tage bei Raumtemperatur in braunen, verschraubten Glasbehältern,
- Anschließend 22 Tage bei Raumtemperatur in Glasbehältern unter Sauerstoffatmosphäre im Dunkeln.

Die letzen 22 Tage der oben beschriebenen Lagerung wurden in Glasphiolen mit einem Volumen von 20 ml durchgeführt. Jeweils 250 mg der vorher bereits für 8 Tage gelagerten Proben wurden dort eingefüllt, die Phiolen wurden mit einem Gummistopfen verschlossen und abgedichtet. Mittels zwei Injektionsnadeln wurde der Kopfraum der Phiolen mit reinem Sauerstoff gespült. Die Proben wurden anschließend im Dunkeln gelagert.

Der Delphinidingehalt der Feststoffe (berechnet als Delphinidinchlorid und angegeben in Gew.-%) wurde mittels der oben beschriebenen HPLC-Methode bestimmt. Die Ergebnisse finden sich in der nachfolgenden Tabelle 3.

| | Zeitablauf [Tage] | | | | |
|---|---|---|---|---|---|
| | Start | 2 | 8 | 19 | 30 |
| Beispiel 3.1 | 1.69 | 1.52 | 1.55 | 1.40 | 0.93 |
| Beispiel 3.2 | 1.30 | 1.20 | 1.14 | 1.03 | 0.68 |
| Beispiel 3.3 | 1. 60 | 1.59 | 1.56 | 1.53 | 1.15 |

Die Ergebnisse zeigen, dass sich erfindungsgemäß ein Delpinidinkomplex herstellen lässt, der selbst unter reiner Sauerstoffatmosphäre eine hohe Stabilität und damit gute Lagerfähigkeit besitzt. Der Komplex besitzt ferner eine gute Löslichkeit in wässrigen, insbesondere leicht sauren Lösungen, so dass sich Delphinidin erfindungsgemäß auf vielfältige Art und Weise formulieren lässt. Die Stabilität des erfindungsgemäßen Feststoffes ist ähnlich gut wie eine Formulierung mit Stärke (Beispiel 3.3), dieses Vergleichsbeispiel lässt sich allerdings nicht in einer wässrigen Lösung formulieren.

### Beispiel 5: Stabilitätsversuche in wässriger Lösung

Zur Bestimmung des Gehalts von Delphinidinchlorid in den delphinidinhaltigen Lösungen wurde ein Reverse Phase HPLC-Verfahren ähnlich dem oben bereits beschriebenen verwendet. Dabei wurden folgende Reagenzien eingesetzt:
Gereinigtes Wasser
Methanol für die Chromatographie
Armeisensäure, p. a.
1 M Salzsäure als volumetrische Lösung.

Als Säule wurde eine Waters X Bridge™ C18, 35 µl, 150 mm x 4,6 mm verwendet.

Die mobilen Phasen waren wie folgt:
Kanal A: Wasser 770 ml, Methanol 230 ml, Armeisensäure 10 ml
Kanal B: Wasser 50 ml, Methanol 950 ml, Armeisensäure 10 ml

Folgendes Gradientenprogramm wurde verwendet:

| Zeit [min] | Prozent Kanal B |
|---|---|
| 0 | 0 |
| 5 | 0 |
| 20 | 20 |
| 25 | 100 |

Stoppzeit: 25 min
Nachlaufzeit (posttime): 8 min
Flussrate: 1 ml/min
Injektionsvolumen: 20 µl
Säulentemperatur: 30°C +/- 2°C
UV-Vis Detektor: 530 µm für den Assay, 275 µm zur Detektion von Verunreinigungen
Integrator: Fläche

### Lösungen und Probenvorbereitung:

Verdünnungslösung 1: Mischung aus 100 ml Methanol und 2,6 ml 1 M HCL
Verdünnungslösung 2: Mischung aus 100 ml 50 %igem Methanol und 2,6 ml 1 M HCL

Kalibrierungslösung: Eine Referenzlösung von Delphinidin wurde durch Einwiegen von 10 mg Delphinidinchlorid in einen 10 ml Kolben und Lösen in Verdünnungslösung 1 hergestellt. Nach dem Lösen wurde ungefähr 10fach verdünnt mit Verdünnungslösung 2 zur Herstellung einer ungefähren Konzentration von 0,1 mg/ml.

Die Kontrollkalibrierungslösung wurde auf die gleiche Art und Weise hergestellt. Die Kalibrierungslösungen wurden sofort mittels HPLC analysiert, da Delphinidinchlorid in Lösung instabil ist.

### Herstellung der Prüflösungen:

Zur Bestimmung des Delphinidingehalts einer erfindungsgemäßen wässrigen Lösung wurden Delphinidin/SBE-ß-CD des Beispiels 3.1 (erfindungsgemäß) und Delphinidin (Vergleichsbeispiel in 0,9 %iger NaCl-Lösung gelöst bis zur Einstellung einer Startkonzentration (bezogen auf das Delphinidin) von 1,584 mg/ml (erfindungsgemäßes Beispiel) bzw. 0,0216 mg/ml (Vergleichsbeispiel). Die Lösungen wurden bei Raumtemperatur hergestellt und anschließend bei 37°C im Dunkeln in verschlossenen Phiolen gelagert.

Nach 1, 2, 3 und 4 h wurde der Delphinidingehalt bestimmt. Die nachfolgende Tabelle gibt den ermittelten Gehalt als Prozentanteil der oben genannten Startkonzentration an.

| Zeit [h] | Delphinidin unkomplexiert | Delphinidin/SBE-ß-CD |
|---|---|---|
| 0 | 100% | 100% |
| 1 | 8,3% | 80,7% |
| 2 | 6,5% | 74,5% |
| 3 | 5,6% | 64,7% |
| 4 | 5,1% | 62,8% |

Die Bestimmung des Delphinidingehalts in den Proben wurde berechnet unter Zuhilfenahme der Agilent ChemStation Software unter Verwendung der Kalibrierung mit den beschriebenen externen Standard.

### II. Wirkung des Anthocyanidin Delphinidin auf Bakterien

### 1. Teststämme und Versuchsaufbau

Es wurden die folgenden Stämme ausgewählt:
- *Pseudomonas aeruginosa* ATCC9027, Biofilm-positives klinisches Isolat aus einer Außenohr-Infektion (Referenzstamm) ;
- *Klebsiella pneumoniae* ATCC700603 Biofilm-positives klinisches Isolat aus Urin von einem ESBL+(Extended Spectrum Beta-Lactamase) -Krankenhauspatienten (Referenzstamm);
- *Staphylococcus aureus* MRSA2318 Biofilm-positives klinisches Isolat aus Trachealsekret vom 22.04.2009 (Uniklinik Würzburg, Neurochirurgische Klinik und Poliklinik I, Intensivstation);
- *Staphylococcus aureus* MRSA2855 Biofilm-positives klinisches Isolat aus Blutkultur vom 22.04.2009(Uniklinik Würzburg, Medizinische Klinik und Poliklinik I, ITS);
- *Staphylococcus aureus* MSSA1155 Biofilm-positives klinisches Isolat vom 27.04.2004 aus Bauchraumabstrich (Uniklinik Würzburg, Urologie, Station B).

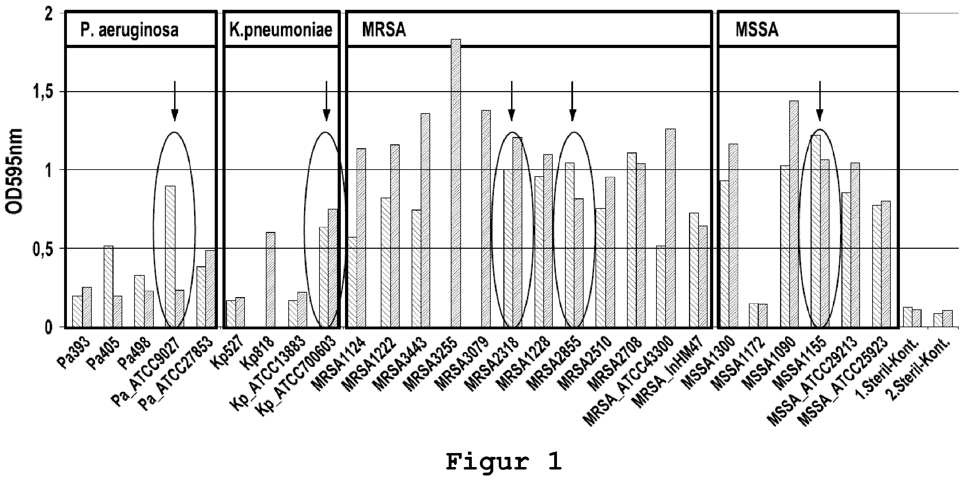

Die vorgenannten Stämme wurden aufgrund ihrer ausgeprägten Biofilmbildung als besonders geeignet für die Untersuchung der Wirkung der erfindungsgemäßen Zusammensetzungen auf Bakterien ausgewählt, wie sich aus Figur 1 ergibt.

Figur 1 zeigt die Ergebnisse einer statischen Biofilmbildung nach 48 Stunden für verschiedene Stämme der Arten *Pseudomonas aeruginosa* und *Klebsiella pneumoniae* sowie MRSA- und MSSA-Stämme in zwei unabhängigen Experimenten. Das Wachstum und die Analyse des Biofilms wurde in einem statischen Modell mittels des Kristallviolett-Tests gemäß dem Basisprotokoll 1 in Current Protocols in Microbiology 1B.1.2 -1B.1.4 "MICROTITER PLATE BIOFILM ASSAY" [publiziert online August 2011 in Wiley Online Library (wileyonlinelib&rary.com) DOI: 10.1002/9780471729259.mc01b01s22] bestimmt.

### 2. Wirkstoffanalyse

Die Untersuchung des Einflusses von Delphinidin auf das bakterielle Wachstum und die bakterielle Biofilmbildung erfolgte mittels
a) der visuellen Bewertung des bakteriellen Wachstums (statisches Modell),
b) Vitalitätstest (Ausstrich auf Blutagarplatten)
c) Analyse des Biofilms in einem statischen Modell mittels des Kristallviolett-Tests wie oben unter Ziffer 1 beschrieben.

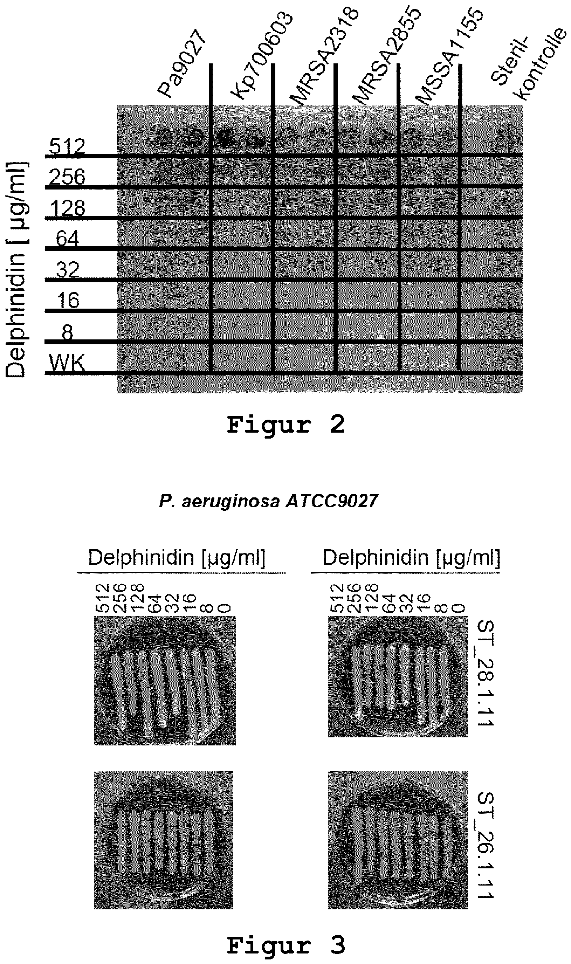

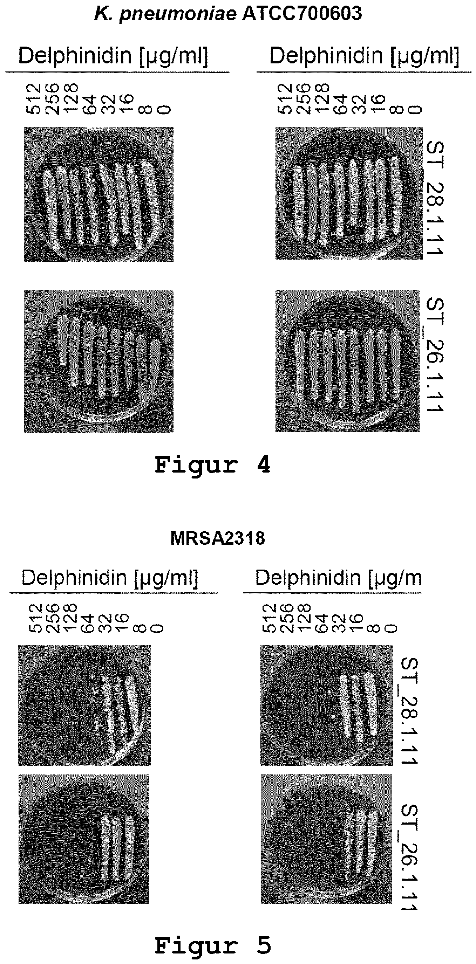

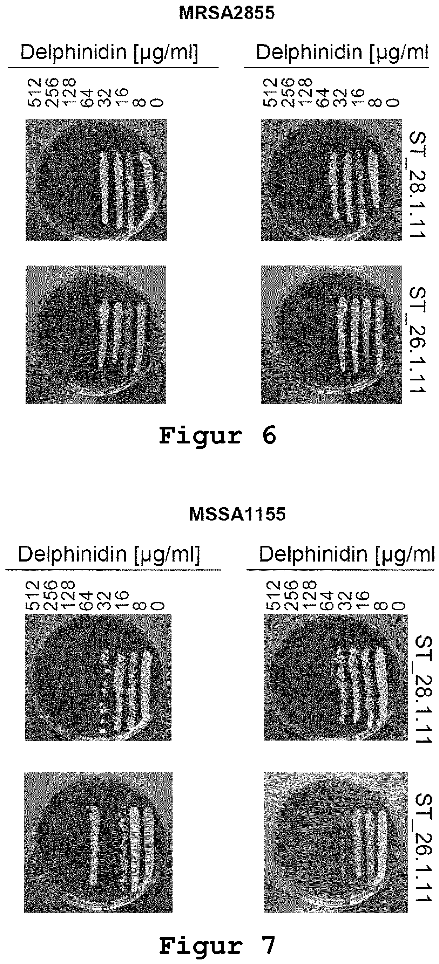

Zuerst wurden 1x10⁶ Bakterien/Well in 100 µl RPMI-1640 Zellmedium mit Phenolrot als Indikator für den pH-Wert (und damit als indirekter Indikator für das bakterielle Wachstum) in einer 96-Well-Polysterol-Zellkulturschale vorgelegt. Als Kontrolle diente steriles RPMI-1640. Anschließend wurde in RPMI-1640 gelöstes Delphinidin aus einer zuvor erstellten Verdünnungsreihe hinzugefügt und die Zellkulturschale für 48 Stunden bei 37°C inkubiert, dessen visuelles Ergebnis in Figur 2 dargestellt ist. Der Farbumschlag von (Phenol-)Rot zu Gelb korreliert mit der Stärke des bakteriellen Wachstums.

Ausstriche der Bakterien aus der Kulturschale sind in den Figuren 3 (*P. aeruginosa* ATCC9027), 4 (*K. pneumoniae* ATCC700603), 5 (MRSA2318), 6 (MRSA2855) und 7 (MSSA1155) dargestellt. "ST_26.1.11" und "ST_28.1.11" bei den Ausstrichen auf den Agarplatten verweist auf das jeweilige Ausstrichdatum der duplizierten Versuche.

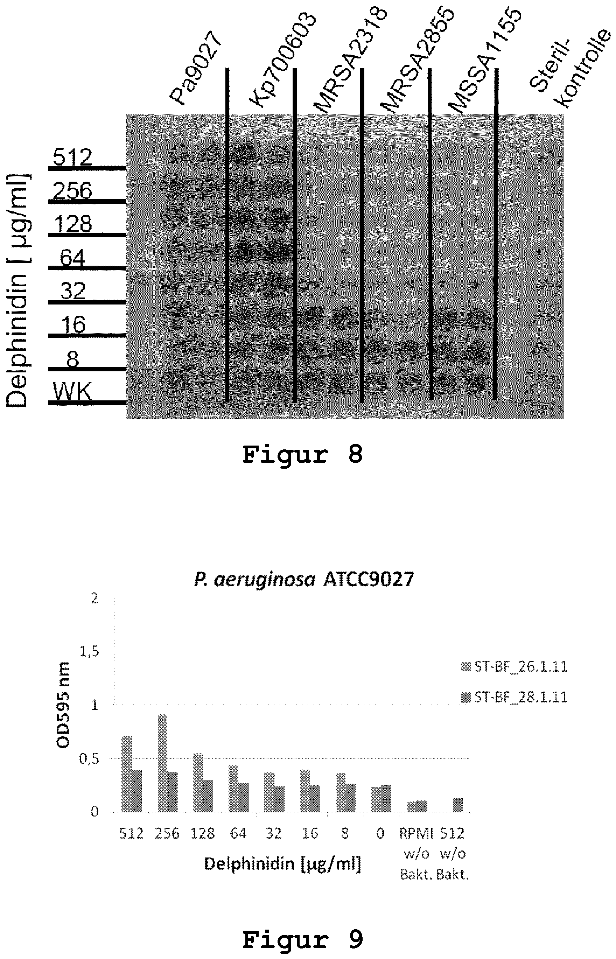

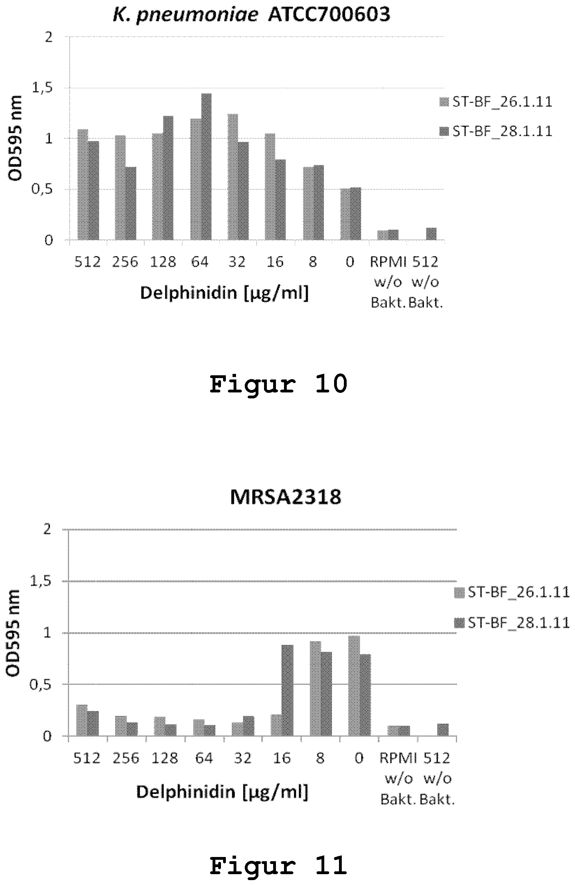

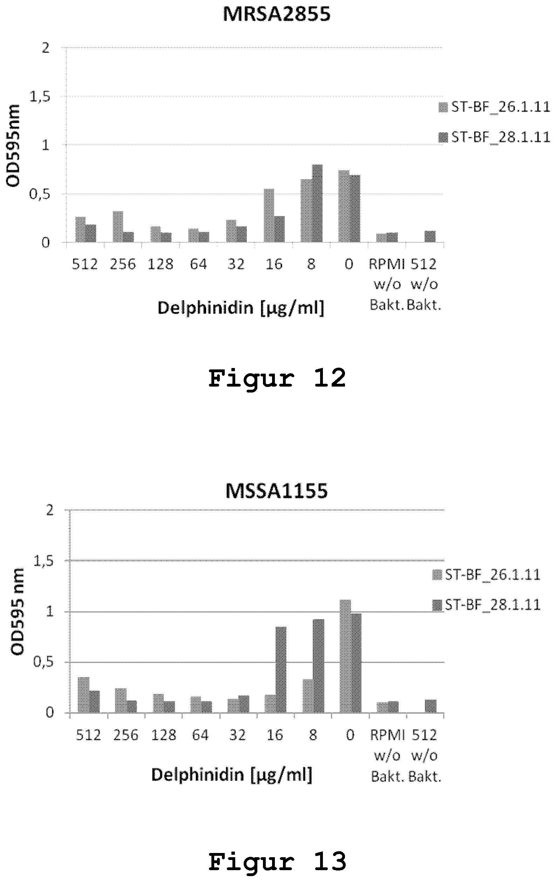

Mit gleichem Versuchsaufbau erfolgte die Analyse des Biofilms mittels des Kristallviolett-Tests wie oben unter Ziffer 1 beschrieben, dessen Ergebnis nach 48 Stunden Inkubation und Kristallviolett-Test in Figur 8 insgesamt, und in den Figuren 9-13 jeweils nach Messung der optischen Dichte graphisch aufbereitet für die jeweiligen Delphinidin-Konzentrationen bei den einzelnen Bakterienstämmen dargestellt sind (Figur 9: *P. aeruginosa* ATCC9027, Figur 10: *K. pneumoniae* ATCC700603, Figur 11: MRSA2318, Figur 12: MRSA2855, Figur 13: MSSA1155).

Die Versuchsergebnisse lassen sich wie folgt zusammenfassen:
- Delphinidin besitzt keinen hemmenden Einfluss auf das Wachstum und die Biofilm-Bildung der gram-positiven Bakterien *P. aeruginosa* und *K. pneumoniae.* Die Biofilm-Bildung scheint im Fall von *K. pneumoniae* sogar noch stimuliert zu werden.
- Dagegen besitzt Delphinidin einen hemmenden Effekt sowohl auf das Wachstum als auch die Biofilm-Bildung von *S. aureus* (sowohl MSSA als auch MRSA). Diese Wirkung nimmt dosisabhängig ab.

## Patentansprüche

1. Verwendung von Delphinidin oder dessen Salze oder eines Komplexes umfassend Delphinidin zur zumindest teilweisen Neutralisation eines mit Bakterien ausgewählt aus der Gruppe bestehend aus Antibiotika-resistenten *Staphylococcus aureus* und Antibiotika-sensitiven *Staphylococcus aureus* kontaminierten Objekts, wobei das Objekt kein lebendes Tier und kein lebender Mensch ist und
wobei besagte zumindest teilweise Neutralisation zu einer zumindest teilweisen Zerstörung oder Auflösung oder Inaktivierung oder Verhinderung der Vermehrung der *Staphylococcus aureus* Bakterien oder einer Verhinderung der *Staphylococcus* aureus-vermittelten Biofilm-Bildung führt.

2. Verwendung nach Anspruch 1, **dadurch gekennzeichnet, dass** die Antibiotika ausgewählt sind aus der Gruppe bestehend aus
- β-Lactam Antibiotika, einschließlich Penicillin, Cephalosporine, Carbapeneme und Monobactame,
- Chinolone
- Tetracycline,
- Aminoglykoside,
- Erythromycin,
- Sulfonamide und
- Vancomycin.

3. Verwendung nach Anspruch 1 oder 2, **dadurch gekennzeichnet, dass** das Delphinidin oder dessen Salze oder der Komplex umfassend Delphinidin in einer wässrigen Lösung oder einem Feststoff enthaltend sind.

4. Verwendung nach Anspruch 3, **dadurch gekennzeichnet, dass** die wässrige Lösung oder der Feststoff ein Zusatz von Werkstoffen oder einer Spüllösung sind.

5. Verfahren zur Behandlung eines Objekts, wobei das Objekt kein lebendes Tier und kein lebender Mensch ist, umfassend:
a) die Bereitstellung
i) von Delphinidin oder dessen Salzen oder eines Komplexes umfassend Delphinidin oder eine wässrige Lösung oder ein Feststoff enthaltend Delphinidin oder dessen Salze oder einen Komplex umfassend Delphinidin und
ii) eines nicht lebenden Objekts, das verdächtig wird, mit Bakterien ausgewählt aus der Gruppe bestehend aus Antibiotika-resistenten *Staphylococcus aureus* und Antibiotika-sensitiven *Staphylococcus aureus* kontaminiert zu sein, und
b) die Behandlung des Objekts mit Delphinidin oder dessen Salzen oder dem Komplex umfassend Delphinidin oder der wässrigen Lösung oder dem Feststoff unter solchen Bedingungen, dass die Bakterien, die verdächtigerweise das Objekt kontaminieren, zumindest teilweise zerstört oder aufgelöst oder inaktiviert werden oder eine *Staphylococcus* aureus-vermittelten Biofilm-Bildung verhindert wird.

6. Verfahren nach Anspruch 5, **dadurch gekennzeichnet, dass** das Objekt ein Tierkörper oder ein Teil davon ist.

7. Verfahren nach Anspruch 6, **dadurch gekennzeichnet, dass** die Tierkörper oder Teile davon ausgewählt sind aus der Gruppe bestehend aus
- Körper eines Rindes oder ein Teil davon,
- Körper eines Schweins oder ein Teil davon,
- Geflügelkörper oder ein Teil davon und
- Körper eines im Wasser lebenden Tieres oder ein Teil davon.

8. Verfahren nach Anspruch 5, **dadurch gekennzeichnet, dass** das Objekt ein Nahrungs- und/oder Futtermittel ist.

9. Verfahren nach Anspruch 8, **dadurch gekennzeichnet, dass** das Objekt ein Nahrungs- und/oder Futtermittel ist, wobei das Nahrungs- und/oder Futtermittel ausgewählt ist aus der Gruppe bestehend aus
- einem Fleischprodukt,
- einem verarbeiteten Fleischprodukt,
- einem Molkereiprodukt,
- Gemüse und deren Teilen und
- Obst und deren Teilen.

10. Verfahren nach Anspruch 5, **dadurch gekennzeichnet, dass** die Objekte ausgewählt sind aus der Gruppe bestehend aus
a) Vorrichtungen und/oder Einrichtungen und/oder Ausrüstungen und/oder Geräten für
- medizinische Anwendungen,
- die Lebensmittelverarbeitung,
- das Militär,
b) Schutzausrüstungen,
c) häusliche Objekte,
d) Gebäudeeinrichtungen und
e) Bauwerke.

11. Nicht-therapeutische Verwendung von Delphinidin oder dessen Salzen oder einem Komplex umfassend Delphinidin oder einer wässrigen Lösung oder eines Feststoffes enthaltend Delphinidin oder dessen Salze oder einen Komplex umfassend Delphinidin in der Behandlung eines Objekts das verdächtigt wird, mit Antibiotika-resistenten oder Antibiotika-sensitiven Bakterien der Art *Staphylococcus aureus* infiziert zu sein.

12. Delphinidin oder dessen Salze oder Komplex umfassend Delphinidin oder wässrige Lösung oder Feststoff enthaltend Delphinidin oder dessen Salze oder einen Komplex umfassend Delphinidin zur Verwendung in der Behandlung oder Prophylaxe von Menschen oder Tieren, die verdächtig sind, mit Antibiotika-resistenten oder Antibiotika-sensitiven Bakterien der Art *Staphylococcus aureus* infiziert zu sein.

13. Delphinidin oder dessen Salze oder Komplex umfassend Delphinidin oder wässrige Lösung oder Feststoff zur Verwendung nach Anspruch 12, **dadurch gekennzeichnet, dass** die Infektion Krankheiten mit Symptomen bedingt, die ausgewählt sind aus der Gruppe bestehend aus:
- Hautinfektionen, einschließlich Furunkel und Karbunkel,
- Pyomyositis,
- Lungenentzündung,
- Endokarditis,
- Toxisches Schock-Syndrom,
- Sepsis und
- Mastitis.

14. Verwendung nach einem der Ansprüche 1-4, Verfahren nach einem der Ansprüche 5-10 oder Verwendung nach Anspruch 11, **dadurch gekennzeichnet, dass** der Komplex umfassend Delphindin
- Sulfoalkylether-β-Cyclodextrin, vorzugsweise ein Sulfobutylether-β-Cyclodextrin umfasst und
- der Substitutionsgrad des Cyclodextrins mit Sulfoalkylethergruppen vorzugsweise 3-8, weiter vorzugsweise 4-8, weiter vorzugsweise 5 bis 8, besonders bevorzugt 6-7 beträgt.

15. Komplex umfassend Delphinidin oder wässrige Lösung oder Feststoff enthaltend einen Komplex umfassend Delphinidin zur Verwendung nach einem der Ansprüche 12 und 13, **dadurch gekennzeichnet, dass** der Komplex umfassend Delphinidin
- Sulfoalkylether-β-Cyclodextrin, vorzugsweise ein Sulfobutylether-β-Cyclodextrin umfasst und
- der Substitutionsgrad des Cyclodextrins mit Sulfoalkylethergruppen vorzugsweise 3-8, weiter vorzugsweise 4-8, weiter vorzugsweise 5 bis 8, besonders bevorzugt 6-7 beträgt.

## Claims

1. Use of delphinidin or salts thereof or of a complex comprising delphinidin for at least partially neutralizing an object contaminated with bacteria selected from the group consisting of antibiotics-resistant *Staphylococcus aureus* and antibiotics-sensitive *Staphylococcus aureus,* the object not being a live animal and not being a live human, and
wherein said at least partial neutralization leads to at least partial destruction or disintegration or inactivation or prevention of reproduction of the *Staphylococcus aureus* bacteria or prevention of *Staphylococcus* aureus-mediated biofilm formation.

2. Use according to Claim 1, **characterized in that** the antibiotics are selected from the group consisting of
- ß-lactam antibiotics, including penicillin, cephalosporins, carbapenems, and monobactams,
- quinolones,
- tetracyclines,
- aminoglycosides,
- erythromycin,
- sulfonamides, and
- vancomycin.

3. Use according to Claim 1 or 2, **characterized in that** the delphinidin or salts thereof or the complex comprising delphinidin are present in an aqueous solution or a solid.

4. Use according to Claim 3, **characterized in that** the aqueous solution or the solid is an addition of materials or of a rinsing solution.

5. Method for treating an object, the object not being a live animal and not being a live human, comprising:
a) providing
i) delphinidin or salts thereof or a complex comprising delphinidin or an aqueous solution or a solid comprising delphinidin or salts thereof or a complex comprising delphinidin, and
ii) an inanimate object suspected of being contaminated with bacteria selected from the group consisting of antibiotics-resistant *Staphylococcus aureus* and antibiotics-sensitive *Staphylococcus aureus,* and
b) treating the object with delphinidin or salts thereof or the complex comprising delphinidin or the aqueous solution or the solid under conditions such that the bacteria suspected of contaminating the object are at least partially destroyed or disintegrated or inactivated, or *Staphylococcus* aureus-mediated biofilm formation is prevented.

6. Method according to Claim 5, **characterized in that** the object is an animal body or a part thereof.

7. Method according to Claim 6, **characterized in that** the animal bodies or parts thereof are selected from the group consisting of
- body of a bovine, or a part thereof,
- body of a pig, or a part thereof,
- poultry body or a part thereof, and
- body of a water-dwelling animal, or a part thereof.

8. Method according to Claim 5, **characterized in that** the object is a food and/or feed.

9. Method according to Claim 8, **characterized in that** the object is a food and/or feed, the food and/or feed being selected from the group consisting of:
- a meat product,
- a processed meat product,
- a dairy product,
- vegetables and parts thereof, and
- fruit and parts thereof.

10. Method according to Claim 5, **characterized in that** the objects are selected from the group consisting of:
a) apparatus and/or installations and/or equipment and/or instruments for
- medical applications,
- food processing,
- the military,
b) protective equipment,
c) domestic objects,
d) building installations, and
e) construction works.

11. Non-therapeutic use of delphinidin or its salts or a complex comprising delphinidin, or of an aqueous solution or of a solid comprising delphinidin or its salts or a complex comprising delphinidin, in the treatment of an object suspected of being infected with antibiotics-resistant or antibiotics-sensitive bacteria of the species *Staphylococcus aureus.*

12. Delphinidin or its salts or complex comprising delphinidin or aqueous solution or solid comprising delphinidin or its salts or a complex comprising delphinidin, for use in the treatment or prophylaxis of humans or animals suspected of being infected with antibiotics-resistant or antibiotics-sensitive bacteria of the species *Staphylococcus aureus.*

13. Delphinidin or its salts or complex comprising delphinidin or aqueous solution or solid, for use according to Claim 12, **characterized in that** the infection causes diseases with symptoms selected from the group consisting of:
- skin infections, including boils and carbuncles,
- pyomyositis,
- pneumonia,
- endocarditis,
- toxic shock syndrome,
- sepsis, and
- mastitis.

14. Use according to any of Claims 1-4, method according to any of Claims 5-10, or use according to Claim 11, **characterized in that** the complex comprising delphinidin
- comprises sulfoalkyl ether-β-cyclodextrin, preferably a sulfobutyl ether-β-cyclodextrin, and
- the degree of substitution of the cyclodextrin by sulfoalkyl ether groups is preferably 3-8, more preferably 4-8, more preferably 5 to 8, very preferably 6-7.

15. Complex comprising delphinidin or aqueous solution or solid comprising a complex comprising delphinidin for use according to either of Claims 12 and 13, **characterized in that** the complex comprising delphinidin
- comprises sulfoalkyl ether-β-cyclodextrin, preferably a sulfobutyl ether-β-cyclodextrin, and
- the degree of substitution of the cyclodextrin by sulfoalkyl ether groups is preferably 3-8, more preferably 4-8, more preferably 5 to 8, very preferably 6-7.

## Revendications

1. Utilisation de delphinidine ou de ses sels ou d'un complexe comprenant de la delphinidine, pour la neutralisation au moins partielle d'un objet contaminé par des bactéries choisies dans le groupe constitué par *Staphylococcus aureus* résistant aux antibiotiques et *Staphylococcus aureus* sensible aux antibiotiques, l'objet n'étant pas un animal vivant ni un être humain vivant et ladite neutralisation au moins partielle conduisant à une au moins partielle destruction ou désintégration ou inactivation ou prévention de la multiplication des bactéries *Staphylococcus aureus* ou à une prévention de la formation de biofilm causé par *Staphylococcus aureus.*

2. Utilisation selon la revendication 1, **caractérisée en ce que** les antibiotiques sont choisis dans le groupe constitué par
- les antibiotiques β-lactame, y compris la pénicilline, les céphalosporines, les carbapénèmes et les monobactames,
- les quinolones,
- les tétracyclines,
- les aminoglycosides,
- l'érythromycine,
- les sulfonamides et
- la vancomycine.

3. Utilisation selon la revendication 1 ou 2, **caractérisée en ce que** la delphinidine ou ses sels ou le complexe comprenant de la delphinidine sont contenus dans une solution aqueuse ou une matière solide.

4. Utilisation selon la revendication 3, **caractérisée en ce que** la solution aqueuse ou la matière solide est un apport de matériaux ou d'une solution de lavage.

5. Procédé pour le traitement d'un objet, l'objet n'étant pas un animal vivant ni un être humain vivant, comprenant :
a) la disposition
i) de delphinidine ou de ses sels ou d'un complexe comprenant de la delphinidine ou d'une solution aqueuse ou d'une matière solide contenant de la delphinidine ou ses sels ou un complexe comprenant de la delphinidine et
ii) d'un objet non vivant qui est suspecté d'être contaminé par des bactéries choisies dans le groupe constitué par *Staphylococcus aureus* résistant aux antibiotiques et *Staphylococcus aureus* sensible aux antibiotiques, et
b) le traitement de l'objet par de la delphinidine ou ses sels ou le complexe comprenant de la delphinidine ou la solution aqueuse ou la matière solide, dans des conditions telles que les bactéries qui sont suspectées contaminer l'objet sont au moins en partie détruites ou désintégrées ou inactivées ou une formation de biofilm causé par *Staphylococcus aureus* est prévenue.

6. Procédé selon la revendication 5, **caractérisé en ce que** l'objet est un corps d'animal ou une partie d'un tel corps.

7. Procédé selon la revendication 6, **caractérisé en ce que** le corps d'animal ou les parties d'un tel corps sont choisis dans le groupe constitué par
- le corps d'un bovin ou une partie d'un tel corps,
- le corps d'un porcin ou une partie d'un tel corps,
- le corps d'une volaille ou une partie d'un tel corps et
- le corps d'un animal vivant dans l'eau ou une partie d'un tel corps.

8. Procédé selon la revendication 5, **caractérisé en ce que** l'objet est un produit alimentaire et/ou un aliment pour animaux.

9. Procédé selon la revendication 8, **caractérisé en ce que** l'objet est un produit alimentaire et/ou un aliment pour animaux, le produit alimentaire et/ou l'aliment pour animaux étant choisi dans le groupe constitué par
- un produit carné,
- un produit carné transformé,
- un produit laitier,
- un légume et ses parties et
- un fruit et ses parties.

10. Procédé selon la revendication 5, **caractérisé en ce que** les objets sont choisis dans le groupe constitué par
a) des dispositifs et/ou des appareillages et/ou des équipements et/ou des appareils pour
- des applications médicales,
- la transformation de produits alimentaires,
- l'armée,
b) des équipements de protection,
c) des objets domestiques,
d) des appareillages pour le bâtiment et
e) des édifices.

11. Utilisation non thérapeutique de delphinidine ou de ses sels ou d'un complexe comprenant de la delphinidine ou d'une solution aqueuse ou d'une matière solide contenant de la delphinidine ou ses sels ou un complexe comprenant de la delphinidine, dans le traitement d'un objet qui est suspecté d'être infecté par des bactéries appartenant à l'espèce *Staphylococcus aureus,* résistantes aux antibiotiques ou sensibles aux antibiotiques.

12. Delphinidine ou ses sels ou complexe comprenant de la delphinidine ou solution aqueuse ou matière solide contenant de la delphinidine ou ses sels ou un complexe comprenant de la delphinidine, pour utilisation dans le traitement ou la prophylaxie d'êtres humains ou d'animaux qui sont suspectés d'être infectés par des bactéries appartenant à l'espèce *Staphylococcus aureus,* résistantes aux antibiotiques ou sensibles aux antibiotiques.

13. Delphinidine ou ses sels ou complexe comprenant de la delphinidine ou solution aqueuse ou matière solide pour utilisation selon la revendication 12, **caractérisés en ce que** l'infection cause des maladies avec symptômes, qui sont choisies dans le groupe constitué par :
- des infections cutanées, y compris furoncle et anthrax,
- la pyomyosite,
- l'inflammation pulmonaire,
- l'endocardite,
- le syndrome de choc toxique,
- le sepsis et
- la mastite.

14. Utilisation selon l'une quelconque des revendications 1 à 4, procédé selon l'une quelconque des revendications 5 à 10 ou utilisation selon la revendication 11, **caractérisés en ce que** le complexe comprenant de la delphinidine
- comprend une sulfoalkyléther-β-cyclodextrine, de préférence une sulfobutyléther-β-cyclodextrine et
- le degré de substitution de la cyclodextrine par des groupes sulfoalkyléther vaut de préférence 3-8, encore mieux 4-8, encore mieux 5 à 8, de façon particulièrement préférée 6-7.

15. Complexe comprenant de la delphinidine ou solution aqueuse ou matière solide contenant un complexe comprenant de la delphinidine, pour utilisation selon l'une quelconque des revendications 12 et 13, **caractérisés en ce que** le complexe comprenant de la delphinidine
- comprend une sulfoalkyléther-β-cyclodextrine, de préférence une sulfobutyléther-β-cyclodextrine et
- le degré de substitution de la cyclodextrine par des groupes sulfoalkyléther vaut de préférence 3-8, encore mieux 4-8, encore mieux 5 à 8, de façon particulièrement préférée 6-7.
